(19) **Europäisches Patentamt / European Patent Office / Office européen des brevets**

(11) **EP 4 338 841 A1**

(12) ## EUROPEAN PATENT APPLICATION

(43) Date of publication:
**20.03.2024 Bulletin 2024/12**

(21) Application number: **23156797.5**

(22) Date of filing: **15.02.2023**

(51) International Patent Classification (IPC):
**B01L 3/00** (2006.01)  **B82Y 5/00** (2011.01)
**C12Q 1/6844** (2018.01)  **G01N 21/64** (2006.01)

(52) Cooperative Patent Classification (CPC):
(C-Sets available)
**B01L 3/50273; B01L 3/502746; B01L 3/50851;
B01L 3/50857;** B01L 2200/0621; B01L 2200/0689;
B01L 2300/0829; B01L 2300/0864;
B01L 2300/0874; B01L 2300/0887; B01L 2300/168;
B01L 2300/1861; B01L 2400/0406;
B01L 2400/0688; B82Y 5/00;  (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.09.2022 KR 20220116058**

(71) Applicant: **Samsung Electronics Co., Ltd.
Suwon-si, Gyeonggi-do 16677 (KR)**

(72) Inventors:
• **Lee, Jae Hong
Suwon-si, Gyeonggi-do (KR)**
• **Jung, Won Jong
Suwon-si, Gyeonggi-do (KR)**
• **Namkoong, Kak
Suwon-si, Gyeonggi-do (KR)**
• **Jang, Hyeong Seok
Suwon-si, Gyeonggi-do (KR)**
• **Kim, Jin Ha
Suwon-si, Gyeonggi-do (KR)**
• **Youn, Hyung Jun
Suwon-si, Gyeonggi-do (KR)**

(74) Representative: **Grünecker Patent- und
Rechtsanwälte
PartG mbB
Leopoldstraße 4
80802 München (DE)**

Remarks:
Amended claims in accordance with Rule 137(2) EPC.

(54) **GENE AMPLIFICATION CHIP, APPARATUS FOR GENE AMPLIFICATION, AND APPARATUS FOR BIO-PARTICLE ANALYSIS**

(57)  A gene amplification chip includes a chamber layer, a cover layer, a bottom layer, an inlet, and an outlet. The chamber layer has a first passage and through holes which are formed on one side of the first passage. The cover layer is disposed on one side of the chamber layer and has a cover channel formed to communicate with the first passage and the through holes, wherein the cover channel, the first passage and the through holes allow passage of liquids in a divided manner. The bottom layer is disposed on another side of the chamber layer and has a bottom channel formed to communicate with the first passage and the through holes. The inlet is formed in the cover layer and communicates with the cover channel. The outlet communicates with any one of the cover channel and the bottom channel.

FIG. 1

EP 4 338 841 A1

(52) Cooperative Patent Classification (CPC): (Cont.)
C12Q 1/6844

C-Sets
C12Q 1/6844, C12Q 2563/107

**Description**

BACKGROUND

**1. Field**

The following description relates to a gene amplification chip, an apparatus for gene amplification, and an apparatus for bio-particle analysis.

**2. Description of the Related Art**

Clinical or environmental samples are analyzed by a series of biochemical, chemical, and mechanical treatment processes. Recently, there has been much interest in developing techniques for diagnosis or monitoring of biological samples. Molecular diagnostic methods based on nucleic acid amplification techniques have excellent accuracy and sensitivity, and thus are increasingly used in various applications, ranging from diagnosis of infectious diseases or cancer to pharmacogenomics, development of new drugs, and the like.

The polymerase chain reaction (PCR) is a technique for nucleic acid amplification, which has been widely used as a core technology in molecular biological diagnostic methods. The PCR is a method of detecting a target nucleic acid by amplifying a specific nucleic acid sequence, in which heating and cooling processes are repeated to activate or inhibit an enzyme for copying nucleic acid sequences. By gene amplification, the PCR can detect the presence of a target DNA sequence in a sample, and research is conducted for simplifying the PCR process to reduce time and provide user convenience, and for manufacturing a PCR system in a compact size for use in point-of-care testing or individual diagnostic testing.

SUMMARY

Apparatuses consistent with the disclosure may include a gene amplification chip including: a chamber layer formed with a first passage and through holes formed on one side of the first passage; a cover layer disposed on one side of the chamber layer, the cover layer formed with a cover channel communicating with the first passage and the through holes of the chamber layer, wherein the cover channel, the first passage and the through holes allow passage of liquids in a divided manner; a bottom layer disposed on another side of the chamber layer, the bottom layer formed with a bottom channel communicating with the first passage and the through holes of the chamber layer; wherein the cover layer is further formed with an inlet communicating with the cover channel; and at least one of the cover layer and the bottom layer is formed with an outlet communicating with any one of the cover channel and the bottom channel.

An embodiment further include an inlet in the cover layer formed to be closer to the plurality of through hole than to the first passage of the chamber layer.

An embodiment may include the first passage and the cover channel are formed, wherein a sample solution flows from the inlet to the cover channel to be stored in the plurality of through holes, and oil having a lower surface tension than the sample solution flows from the inlet to the cover channel to the first passage and to the bottom channel.

An embodiment may include the first passage having a larger cross-section than a cross-section of a cover channel region connected to the inlet.

An embodiment may include the cross-section of the first passage being defined as a value obtained by multiplying a passage width by a passage length, and the cross-section of the cover channel region is defined as a value obtained by multiplying a channel height by a channel length, the passage length and the channel length are a same length in a same direction, and the passage width is greater than the channel height.

An embodiment may include the cover channel having a larger cross-section than a cross-section of the through holes.

An embodiment may include a cross-section of the through holes having any one of a hexagonal shape, a tetragonal shape, a triangular shape, and a circular shape.

An embodiment may include the chamber layer further a second passage formed on a side opposite the first passage with the through holes formed therebetween.

An embodiment may include the outlet formed to face the second passage.

An embodiment may include the outlet formed in the cover layer.

An embodiment may include the outlet formed in the bottom layer.

An embodiment may include the chamber layer, the cover layer, and the bottom layer that are separately formed and subsequently joined together.

An embodiment may include the chamber layer and the bottom layer that are integrally formed with each other to be joined to the cover layer.

An embodiment may include each of the chamber layer, the cover layer, and the bottom layer being made of any one of silicon, metal, glass, and polymer.

An embodiment may include the cover layer being made of a transparent material.

An embodiment may include the first passage and the plurality of through holes in the chamber layer are separated by a non-through area, and cross sectional dimensions of the cover channel and the first passage are different from each other to flow liquid from the inlet through one of the first passage and the cover channel.

An embodiment may include a gene amplification chip comprising a chamber layer formed with a first passage and through holes formed on one side of the first passage, a cover layer disposed on one side of the chamber layer, the cover layer being formed with a cover channel communicating with the first passage and the through holes of the chamber layer, wherein the cover channel, the first passage and the through holes allow passage of a sample solution in a divided manner, a bottom layer disposed on another side of the chamber layer, the bottom layer formed with a bottom channel communicating with the first passage and the through holes of the chamber layer, wherein the cover layer is further formed with an inlet communicating with the cover channel, at least one of the cover layer and the bottom layer is formed with an outlet communicating with any one of the cover channel and the bottom channel; a heat source configured to heat the gene amplification chip to allow gene amplification to occur in the sample solution; and a heat source controller configured to control temperature and time of heating by the heat source.

An embodiment may include the heat source includes a light source configured to emit light; and a photothermal film attached to an outer surface of the bottom layer and configured to convert light, emitted by the light source, into heat to heat the gene amplification chip.

An embodiment may include the heat source includes a thermoelement.

An embodiment may include an apparatus for gene amplification in which gene amplification of a sample solution occurs; a detector configured to detect a signal generated in response to the occurrence of gene amplification of the sample solution; and a processor configured to analyze bio-particles based on the detected signal, wherein the apparatus for gene amplification may include: a gene amplification chip comprising a chamber layer formed with a first passage and through holes formed on one side of the first passage, a cover layer disposed on one side of the chamber layer, the cover layer formed with a cover channel communicating with the first passage and the through holes of the chamber layer, wherein the cover channel, the first passage and the through holes allow passage of the sample solution in a divided manner, a bottom layer disposed on another side of the chamber layer, the bottom layer formed with a bottom channel formed communicating with the first passage and the through holes of the chamber layer, wherein the cover layer is further formed with an inlet formed communicating with the cover channel, and at least one of the cover layer and the bottom layer is formed with an outlet communicating with any one of the cover channel and the bottom channel; a heat source configured to heat the gene amplification chip to allow gene amplification to occur in the sample solution; and a heat source controller configured to control temperature and time of heating by the heat source.

An embodiment may include a processor calculating a concentration of the sample solution by measuring a number of through holes in which a fluorescence signal is observed.

BRIEF DESCRIPTION OF THE DRAWINGS

FIG. 1 is a perspective view of a gene amplification chip according to an embodiment of the present disclosure.

FIG. 2 is an exploded perspective view of FIG. 1.

FIG. 3 is a plan view of FIG. 1.

FIG. 4 is a cross-sectional view taken along line 4-4 of FIG. 3.

FIG. 5 is a cross-sectional view taken along line 5-5 of FIG. 3.

FIG. 6 is a cross-sectional view taken along line 6-6 of FIG. 3.

FIGS. 7A to 7C are diagrams explaining a working principle of a gene amplification chip.

FIGS. 8A to 8G are diagrams illustrating an example of filling a gene amplification chip with a sample solution and oil.

FIGS. 9A to 9D are diagrams illustrating another example of filling a gene amplification chip with a sample solution.

FIGS. 10A to 10D are diagrams illustrating yet another example of filling a gene amplification chip with a sample solution and oil.

FIG. 11 is a cross-sectional view of an example of an outlet formed in a bottom layer.

FIG. 12 is a cross-sectional view of an example of a chamber layer from which a second passage is omitted.

FIG. 13 is a block diagram illustrating an apparatus for gene amplification according to an embodiment of the present disclosure.

FIG. 14 is a block diagram illustrating an apparatus for gene amplification according to another embodiment of the present disclosure.

FIG. 15 is a block diagram illustrating an apparatus for gene amplification including a heat source according to another embodiment of the present disclosure.

FIG. 16 is a block diagram illustrating an apparatus for bio-particle analysis according to an embodiment of the present disclosure.

FIG. 17 is a block diagram illustrating an apparatus for bio-particle analysis according to another embodiment of the present disclosure.

FIG. 18 is a flowchart illustrating an example of a method of bio-particle analysis.

Throughout the drawings and the detailed description, unless otherwise described, the same drawing reference numerals will be understood to refer to the same elements, features, and structures. The relative size and depiction of these elements may be exaggerated for clarity, illustration, and convenience.

DETAILED DESCRIPTION

Details of other embodiments are included in the following detailed description and drawings. Advantages and features of example embodiment, and a method of achieving the same will be more clearly understood from the following embodiments described in detail with reference to the accompanying drawings. Throughout the drawings and the detailed description, unless otherwise described, the same drawing reference numerals will be understood to refer to the same elements, features, and structures.

It will be understood that, although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'unit' or 'module', etc., should be understood as a unit that performs at least one function or operation and that may be embodied as hardware, software, or a combination thereof.

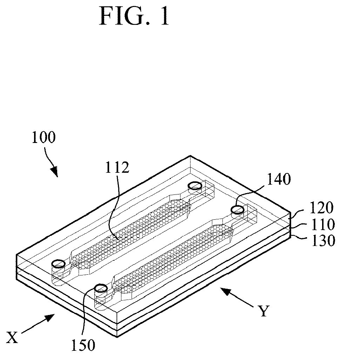

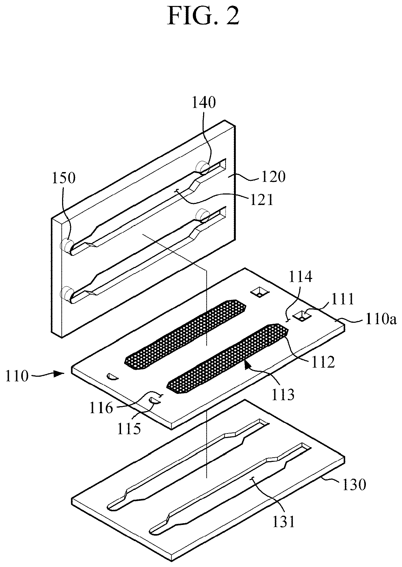

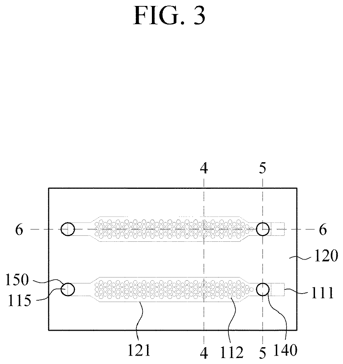

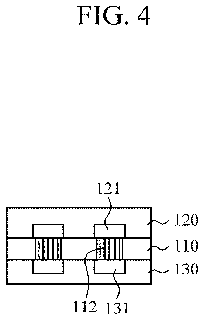

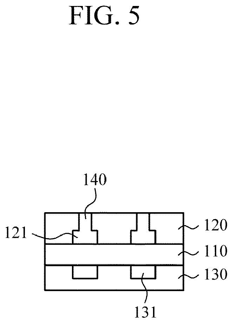

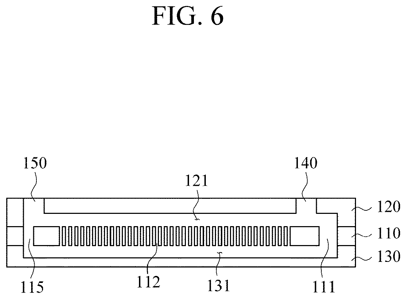

FIG. 1 is a perspective view of a gene amplification chip according to an embodiment of the present disclosure. FIG. 2 is an exploded perspective view of FIG. 1. FIG. 3 is a plan view of FIG. 1. FIG. 4 is a cross-sectional view taken along line 4-4 of FIG. 3. FIG. 5 is a cross-sectional view taken along line 5-5 of FIG. 3. FIG. 6 is a cross-sectional view taken along line 6-6 of FIG. 3.

Referring to FIGS. 1 to 6, a gene amplification chip 100 may include a chamber layer 110, a cover layer 120, a bottom layer 130, an inlet 140, and an outlet 150.

The chamber layer 110 may include a first passage 111, and through holes 112 which are formed on one side of the first passage 111 and in which a sample solution is stored in a divided manner. The sample solution is divided among the through holes. In addition, as will be explained later, the sample solution may flow through the gene amplification chip by taking different flow paths. The chamber layer 110 may have a structure in which the first passage 111 and the through holes 112 are formed in a chamber base 110a. The chamber layer 110 may be formed in such a manner that with respect to the chamber base 110a disposed horizontally and parallel to the other layers, the first passage 111 is formed by vertically passing through the chamber base 110a, and the through holes 112 are also formed by vertically passing through the chamber base 110a.

The chamber base 110 may have a constant thickness. The first passage 111 may be formed at an edge of one side of the chamber base 1 10a. The first passage 111 may be formed with a constant cross-section by passing through the chamber base 110a. The first passage 111 may be formed in various shapes, such as a tetragon, a circle, and the like. That is, the first passage 111 may be formed in the shape of a tetragonal column, a circular column, and the like. The first passage 111 may be formed so that oil injected through the inlet 140 may pass through the first passage 111 to flow to a bottom channel 131 of the bottom layer 130.

The through holes 112 may be arranged in a group on one side of the first passage 111 to form a through-hole array 113. The through-hole array 113 may be disposed at the center of the chamber base 110a. The chamber base 110a may have a first non-through hole area 114 between the first passage 111 and the through-hole array 113. The through-hole array 113 may be spaced apart from the first passage 111 with the first non-through hole area 114 therebetween. The through holes 112 may be arranged at regular intervals in the X-axis and Y-axis directions. The through holes 112 may be arranged longer or shorter in the X-axis direction than in the Y-axis direction, or may be arranged in equal length in the X-axis direction than in the Y-axis direction, but the through holes 112 are not limited thereto.

All the through holes 112 may have the same size. The respective through holes 112 may be formed with a constant cross-section by passing through the chamber base 110a. The cross-section of the through holes 112 may have various shapes, such as hexagon, tetragon, triangle, circle, and the like. That is, the through holes 112 may be formed in the shape of a hexagonal column, a tetragonal column, a circular column, and the like.

Dozens of through holes 112 may be formed. The through holes 112 may have a volume of 10 pL or more. The number and volume of the through holes 112 are not limited thereto. The through holes 112 are formed so that a sample solution flowing through the inlet 140 to a cover channel 121 of the cover layer 120 may be contained therein in a divided manner.

The chamber layer 110 may further include a second passage 115 formed on a side opposite the first passage 111 with the through holes 112 formed therebetween. The through-hole array 113 may be disposed between the first passage 111 and the second passage 115. The chamber base 110a may include a second non-through hole area 116 between the second passage 115 and the through-hole array 113. The through-hole array 113 may be spaced apart from the second passage 115 with the second non-through hole area 116 therebetween.

With respect to the chamber base 110a disposed horizontally and parallel to the other layers, the second passage 115 may be formed by vertically passing through the chamber base 110a. The second passage 115 may be formed with a constant cross-section by passing through the chamber base 1 10a. The second passage 115 may have the cross-section of various shapes, such as tetragon, circle, and the like. That is, the second passage 115 may be formed in the shape of a tetragonal column, a circular column, and the like. The second passage 115 may allow oil to flow between the cover channel 121 of the cover layer 120 and the bottom channel 131 of the bottom layer 130.

The cover layer 120 may include the cover channel 121 formed to communicate with the first passage 111 and the through holes 112, and the cover layer 120 may be formed on one side of the chamber layer 110. The cover layer 120 may have the cover channel 121 formed on a lower surface thereof. The cover layer 120 may be formed over the chamber layer 110 while the cover channel 121 is directed toward the chamber layer 110.

The cover channel 121 may be formed in a recessed shape extending from the first passage 111 to the second passage 115 and along the through-hole array 113. With respect to the cover layer 120 disposed horizontally and parallel to the other layers, the cover channel 121 may be formed to have a predetermined height from the first passage 111 to the second passage 115.

A region of the cover channel 121, which extends from the first passage 111 to the first non-through hole area 114, may have the same length in the Y-axis direction as a length of the first passage 111 in the Y-axis direction. A region of the cover channel 121, which extends from the second passage 115 to the second non-through hole area 116, may have the same length in the Y-axis direction as a length of the second passage 115 in the Y-axis direction.

The cover channel 121 may be formed so that a sample solution injected through the inlet 140 may be stored in the through holes 112. After the sample solution is stored in the through holes 112, the cover channel 121 may allow the oil, injected through the inlet 140, to be filled therein such that an upper opening of the through holes 112 may be sealed by the oil. The cover channel 121 may have a larger cross-section than the cross-section of the through holes 112, thereby allowing the sample solution and the oil to flow smoothly.

The bottom layer 130 may include the bottom channel 131 formed to communicate with the first passage 111 and the through holes 112, and may be formed on a side of the chamber layer 110 opposite the cover layer 120. The bottom layer 130 may have the bottom channel 131 formed on an upper surface thereof. The bottom layer 130 may be formed under the chamber layer 110 with the bottom channel 131 facing toward the chamber layer 110.

The bottom channel 131 may be formed in a recessed shape extending from the first passage 111 to the second passage 115 and along the through-hole array 113. With respect to the bottom layer 130 disposed horizontally and parallel to the other layers, the bottom channel 131 may be formed to have a predetermined height from the first passage 111 to the second passage 115.

A region of the bottom channel 131, which extends from the first passage 111 to the first non-through hole area 114, may have the same length in the Y-axis direction as a length of the first passage 111 in the Y-axis direction. A region of the bottom channel 131, which extends from the second passage 115 to the second non-through hole area 116, may have the same length in the Y-axis direction as the length of the second passage 115 in the Y-axis direction. The bottom channel 131 may have the same shape as the cover channel 121.

After the sample solution is stored in the through holes 112, the bottom channel 131 may allow the oil, injected through the inlet 140, to be filled therein such that a lower opening of the through holes 112 may be sealed by the oil. The bottom channel 131 may have a cross-section of a size sufficient to allow the oil to flow smoothly.

The inlet 140 may be formed in the cover layer 120 to communicate with the cover channel 121. The inlet 140 may be connected to the cover channel 121 with a length equal to or shorter than the cover channel 121 in the Y-axis direction, but the length thereof is not limited thereto. With respect to the cover layer 120 disposed horizontally and parallel to the other layers, the inlet 140 may be formed with a constant cross-section by vertically passing through the cover layer 120. However, the inlet 140 may have an irregular cross-section having a dimension which gradually increases or decreases toward the cover channel 121, etc., but is not limited thereto. The inlet 140 may be formed in various shapes, such as a circle, a tetragon, and the like.

The inlet 140 may allow the sample solution and the oil to be injected into the cover channel 121. The sample solution may be one or a duplex of two or more of ribonucleic acid (RNA), deoxyribonucleic acid (DNA), peptide nucleic acid (PNA), and locked nucleic acid (LNA), oligopeptide, protein, toxin, etc., but is not limited thereto.

In addition, the sample solution may be bio-fluids, including at least one of respiratory secretions, blood, urine, perspiration, tears, saliva, etc., or a swab sample of the upper respiratory tract, or a solution of the bio-fluid or the swab sample dispersed in other medium. In this case, the other medium may include water, saline solution, alcohol, phosphate buffered saline solution, vital transport media, etc., but is not limited thereto.

The oil may prevent evaporation of the sample solution stored in the through holes 112 during thermal cycling, as well as contact between the sample solution and gas. The oil may have a lower surface tension than the sample solution.

The inlet 140 may be disposed closer to the through holes 112 than to the first passage 111. The inlet 140 may be disposed to face the non-through hole area 114 between the first passage 111 and the through-hole array 113. The sample solution injected through the inlet 140 may be adhered to the non-through hole area 114, thereby preventing the sample solution from flowing directly to the first passage 111.

The sample solution and the oil may be injected into the cover channel 121 through the inlet 140 by an active/passive driving device. Here, the active/passive driving device may include a passive vacuum void pump, a syringe pump, a vacuum pump, a pneumatic pump, etc., but is not limited thereto.

The sample solution may be filled in the through holes 112 after being subjected to a pretreatment process, such as heating, chemical treatment, treatment with magnetic beads, solid phase extraction, treatment with ultrasonic waves, and the like.

The outlet 150 may be formed to communicate with any one of the cover channel 121 and the bottom channel 131. For example, the outlet 150 may be formed in the cover layer 120 to communicate with the cover channel 121. The outlet 150 may be connected to the cover channel 121 with the same length as the cover channel 121 in the Y-axis direction. With respect to the cover layer 120 disposed horizontally and parallel to the other layers, the outlet 150 may be formed with a constant cross-section by vertically passing through the cover layer 120. The outlet 150 may be formed in various shapes, such as a circle, a tetragon, and the like.

The outlet 150 may be disposed to face the second non-through hole area 116. When the sample solution remains in the cover channel 121 after being filled in the through holes 112, the remaining sample solution may be smoothly discharged from the second non-through hole area 116 to the outlet 150. The remaining sample solution may be removed by capillary drainage and the like through the outlet 150.

The outlet 150 may be disposed to face the second passage 115. The outlet 150 may be disposed at a position corresponding to a region from the second non-through region 116 to the second passage 115. The oil flowing to the bottom channel 131 may pass through the second passage 115 to be discharged smoothly through the outlet 150.

Each of the chamber layer 110, the cover layer 120, and the bottom layer 130 of the gene amplification chip 100 may be made of a material, such as silicon, metal, glass, polymer, and the like.

However, the chamber layer 110, the cover layer 120, and the bottom layer 130 are not limited thereto, and may also be made of materials including an inorganic matter, such as ceramic, graphite, etc., acrylic material, polyethylene terephthalate (PET), polycarbonate, polystylene, polypropylene, and the like. The cover layer 120 may be made of a transparent material, such as glass and the like, so as to have light-transmitting properties.

The chamber layer 110, the cover layer 120, and the bottom layer 130 may be separately formed and subsequently joined together. For example, the chamber layer 110 and the bottom layer 130 may be made of a silicon material separately and may be joined by fusion bonding, and the cover layer 120 may be made of a glass material and may be joined by anodic bonding to the chamber layer 110. Alternatively, successive two layers of the chamber layer 110, the cover layer 120, and the bottom layer 130 may be integrally formed with each other to be joined to a remaining layer. For example, the chamber layer 110 and the bottom layer 130 may be integrally formed with each other and then joined to the cover layer 120.

A sum of thicknesses of the chamber layer 110 and the bottom layer 130 may be 1 mm or less, but is not limited thereto. In consideration of thermal conductivity of materials used for the chamber layer 110 and the bottom layer 130, a total thickness or respective thicknesses of the chamber layer 110 and the bottom layer 130 may be changed.

The chamber layer 110, the cover layer 120, and the bottom layer 130 may be made of materials having different thermal conductivities. For example, thermal conductivities of the materials used for the layers may gradually increase in the order of the cover layer 120, the chamber layer 112, and the bottom layer 130. Alternatively, the chamber layer 110 and the bottom layer 130 may be made of a material having relatively higher thermal conductivity than the cover layer 120. The chamber layer 110, the cover layer 120, and the bottom layer 130 may be made of various materials as long as the materials effectively transfer heat to the sample solution in the through holes 112.

When the sample solution is filled in the through holes 112 of the chamber layer 110, gene amplification may occur in the chamber layer 110. In this case, reverse transcription of an RNA sample may be performed in the chamber layer 110 by using a reverse transcriptase. The gene amplification may include, for example, a nucleic acid amplification reaction including at least one of polymerase chain reaction (PCR) amplification and isothermal amplification, a redox reaction, a hydrolytic reaction, and the like.

The chamber layer 110 may include a plurality of through-hole arrays 113 and may include the first and second passages 111 and 115 provided corresponding to the number of through-hole arrays 113. The cover layer 120 may also include the cover channel 121, the inlet 140, and the outlet 150 which are provided corresponding to the number of through-hole arrays 113. The bottom layer 130 may also include the bottom channel 131 provided corresponding to the number of through-hole arrays 113.

Accordingly, the gene amplification chip 100 may include a plurality of sets of the through-hole arrays 113, the first and second passages 111 and 115, the inlet 140, and the outlet 150. The gene amplification chip 100 may perform the amplification reaction of different sample solutions stored in the plurality of through-hole arrays 113 at the same time, thereby improving efficiency.

The first passage 111 and the cover channel 121 of the gene amplification chip 100 may be formed so that after the sample solution flows from the inlet 140 to the cover channel 121 to be stored in the through holes 112 in a divided manner, oil having a lower surface tension than the sample solution may flow from the inlet 140 to the cover channel 121, the first passage 111, and the bottom channel 131 to be filled therein.

For example, the first passage 111 may have a greater cross-section than a cross-section of the cover channel 121 connected to the inlet 140. Here, when the cross-section of the first passage 111 is defined as a value obtained by multiplying a passage width by a passage length, and the cross-section of the cover channel 121 is defined as a value obtained by multiplying a channel height by a channel length, the passage length and the channel length may be the same length in the same direction, and the passage width may be greater than the channel height.

Accordingly, after flowing from the inlet 140 to the first non-through hole area 114, the sample solution having a higher surface tension than the oil does not flow to the first passage 111 but flows along the cover channel 121 from the first non-through hole area 114 to the through-hole array 113 to be filled in the through holes 112.

In addition, the second passage 115 may have a greater cross-section than the cross-section of the cover channel 121 connected to the outlet 150. Without flowing from the second non-through hole area 116 to the second passage 115, a remaining sample solution may be discharged through the outlet 150.

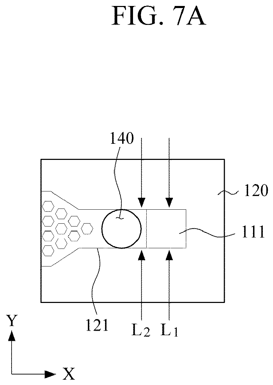

FIGS. 7A to 7C are diagrams explaining a working principle of a gene amplification chip. Here, the cross-section of the first passage 111 may be defined as a value obtained by multiplying a passage width $D$ by a passage length $L_1$, and the cross-section of the cover channel 121 may be defined as a value obtained by multiplying a channel height $h$ by a channel length $L_2$.

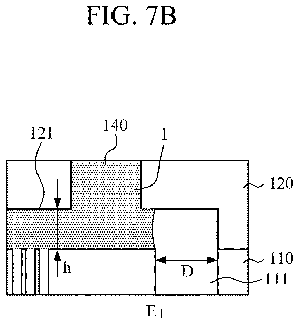

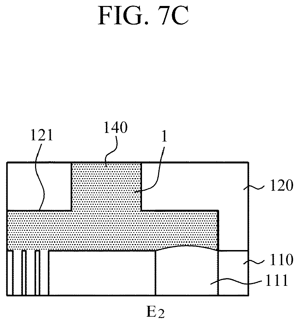

As illustrated in FIG. 7A, the passage length $L_1$ and the channel length $L_2$ may be the same length $L$ in the Y direction. $L_1 = L_2 = L$ FIG. 7B illustrates a state in which a liquid l is injected through the inlet 140 and flows along the cover channel 121 to a boundary between the first non-through hole area 114 and the first passage 111. FIG. 7C illustrates a state in which the liquid l is injected through the inlet 140 and flows along the cover channel 121 from the first non-through hole area 114 to an upper side of the first passage 111.

In order for the liquid l to proceed from the state of FIG. 7B to the state of FIG. 7C, surface energy should decrease. For example, the surface energy is defined as $E_1$ in the state of FIG. 7B, and the surface energy is defined as $E_2$ in the state of FIG. 7C. If $\Delta E = E_1 - E_2 < 0$ is satisfied, the liquid 1 moves from the state of FIG. 7B to the state of FIG. 7C. The Equation regarding $E_1$, $E_2$, and $\Delta E$ may be expressed as follows.

$$E_1 \simeq (\sigma h + \sigma_{sg}(D+h))L$$

$$E_2 \simeq (\sigma D + \sigma_{sl}(D+h))L$$

$$\Delta E = E_2 - E_1 \simeq (\sigma(D-h) - (\sigma_{sg} - \sigma_{sl})(D+h))L$$

Herein, $\sigma$ denotes the surface tension of the liquid, $h$ denotes the channel height of the cover layer, $D$ denotes the passage width of the chamber layer, $L$ denotes the channel length of the cover layer or the passage length of the chamber layer, $\sigma_{sg}$ denotes the surface tension between a wall surface of the cover layer and air, $\sigma_{sl}$ denotes the surface tension between the wall surface of the cover layer and the liquid.

If the passage width $D$ of the chamber layer is smaller than the channel height $h$ of the cover layer, $\Delta E$ is smaller than zero, such that the liquid l passes through the first passage 111 to flow to the bottom channel 131. If the passage width $D$ of the chamber layer is greater than the channel height $h$ of the cover layer, the flow of the liquid l may be determined by the surface tension $\sigma$ of the liquid. A liquid, having a low surface tension $\sigma$ such that $\Delta E$ is a negative number, may pass through the first passage 111 to flow to the bottom channel 131. A liquid, having a high surface tension $\sigma$ such that $\Delta E$ is a positive number, may flow only to the cover channel 121 without passing through the first passage 111.

Generally, the sample solution has a surface tension of approximately 70 mN/m, and the oil has a surface tension of approximately 30 mN/m. Accordingly, based on the above principle, if the passage width $D$ is set to a greater value than the channel height $h$ of the cover layer, the sample solution having a higher surface tension may flow only to the cover channel 121 without passing through the first passage 111, and the oil having a lower surface tension may pass through the first passage 111 to flow to the bottom channel 131. For example, if the channel height $h$ of the cover layer is set to approximately 100 μm, the passage width $D$ of the chamber layer may be set to approximately 700 μm.

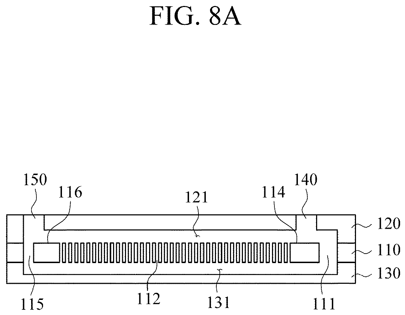

FIGS. 8A to 8G are diagrams illustrating an example of filling a gene amplification chip with a sample solution and oil.

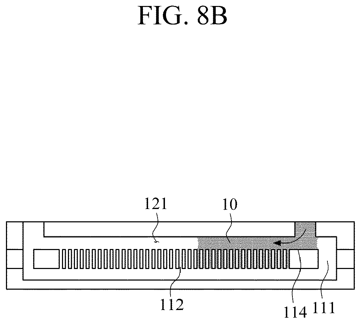

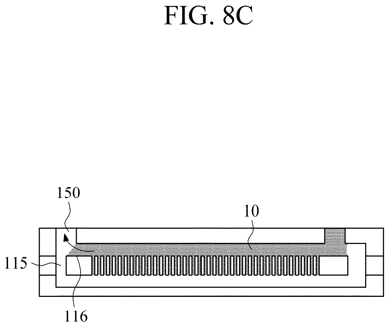

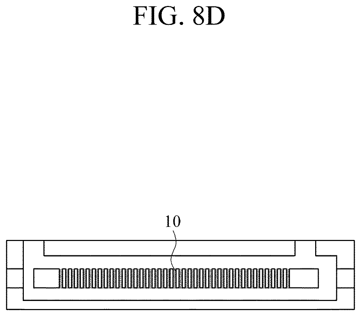

As illustrated in FIGS. 8A and 8B, a sample solution 10 is injected into the cover channel 121 through the inlet 140. Then, the sample solution 10 is adhered to the first non-through hole area 114, and then flows along the cover channel 121 from the first non-through hole area 114 to the through-hole array 113 without flowing to the first passage 111, so as to be filled in the through holes 112. Then, as illustrated in FIG. 8C, the sample solution 10 flows along the cover channel 121 to be filled in all of the through holes 112. Subsequently, as illustrated in FIG. 8D, a remaining sample solution 10, not filled in the through holes 112, is removed from the second non-through hole area 116 through the outlet 150.

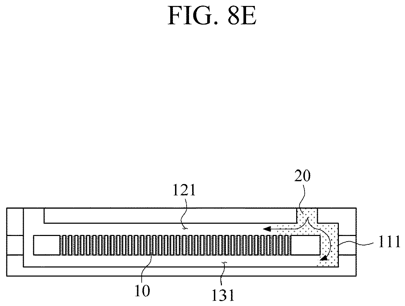

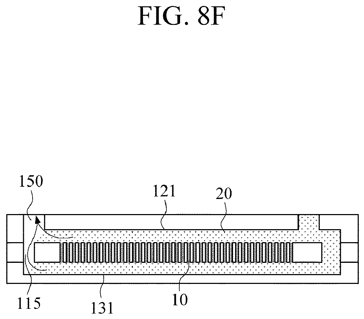

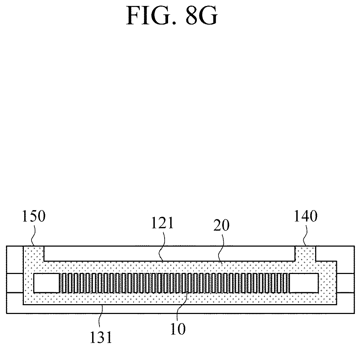

Next, as illustrated in FIG. 8E, oil 20 is injected into the cover channel 121 through the inlet 140. Then, some of the oil 20 starts to be filled in the cover channel 121, and a remaining oil 20 flows through the first passage 111 to be filled in the bottom channel 131. Then, as illustrated in FIGS. 8F and 8G, after be filled in the bottom channel 131, the oil 20 flows to the cover channel 121 by passing through the second passage 115, and then joins the oil 20 filled in the cover channel 121 to reach the outlet 150. Then, the outlet 150 and the inlet 140 may be sealed.

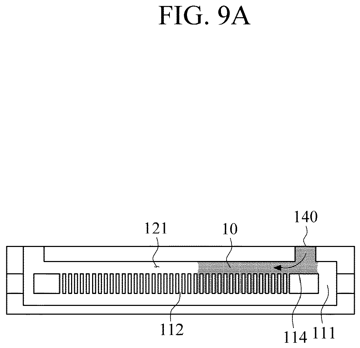

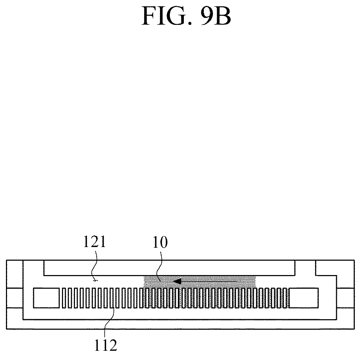

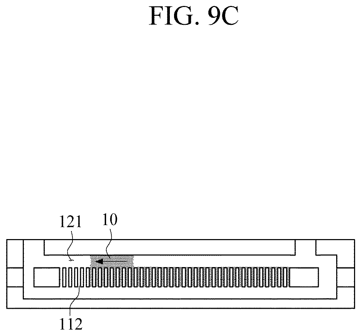

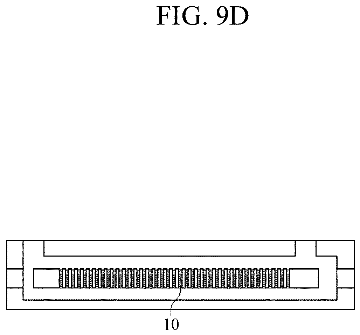

FIGS. 9A to 9D are diagrams illustrating another example of filling a gene amplification chip with a sample solution.

As illustrated in FIGS. 9A to 9D, the sample solution 10 is injected with a volume, corresponding to a total volume of the through holes 112, into the cover channel 121 through the inlet 140. Then, the sample solution 10 may flow from the first non-through hole area 114 to the through-hole array 113 along the cover channel 121 to be sequentially filled in the through holes 112. Subsequently, the oil is filled in the cover channel 121 and the bottom channel 131 through the inlet 140.

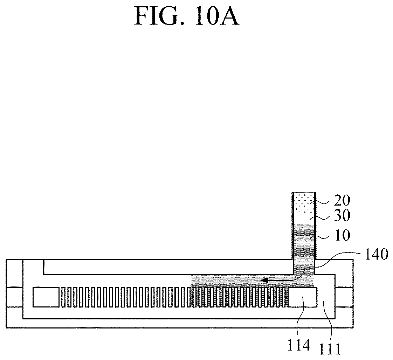

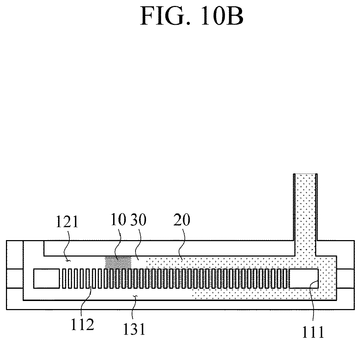

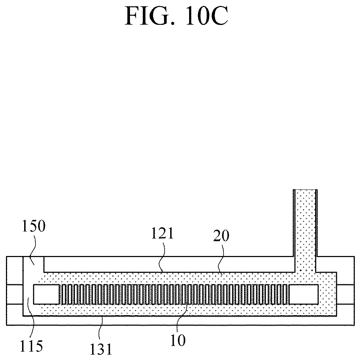

FIGS. 10A to 10D are diagrams illustrating yet another example of filling a gene amplification chip with a sample solution and oil.

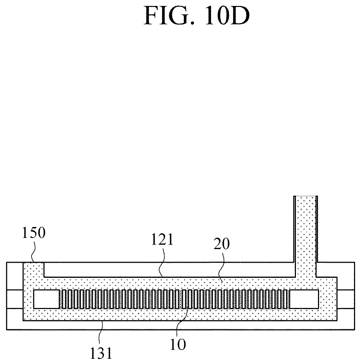

As illustrated in FIGS. 10A to 10C, the sample solution 10, air 30, and the oil 20 are sequentially injected into the cover channel 121 through the inlet 140. Then, the sample solution 10 is adhered to the first non-through hole area 114, and then is pushed by the air 30 and the oil 20 to flow to the through-hole array 113 along the cover channel 121 to be filled in all the through-holes 112. As illustrated in FIG. 10D, the oil 20 continues to flow to the cover channel 121 and the bottom channel 131 to be filled in both the cover channel 121 and the bottom channel 131.

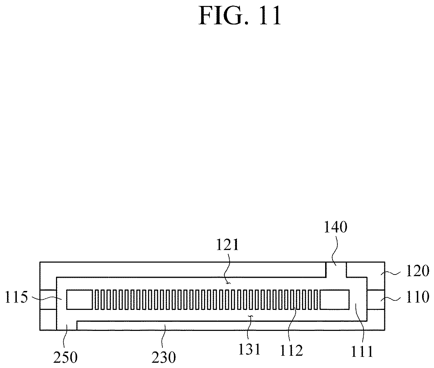

In another embodiment, as illustrated in FIG. 11, an outlet 250 may be formed in a bottom layer 230. In this case, the sample solution may be injected into the cover channel 121 with a volume, corresponding to a total volume of the through holes 112, into the cover channel 121 through the inlet 140, to be filled in all the through holes 112. Then, when the oil is injected into the cover channel 121 through the inlet 140, some of the oil starts to be filled in the cover channel 121, and a remaining oil starts to be filled in the bottom channel 131 by passing through the first passage 111. The oil filled in the cover channel 121 passes through the second passage 115 to flow to the bottom channel 131, and then joins the oil filled in the bottom channel 131 to reach the outlet 250.

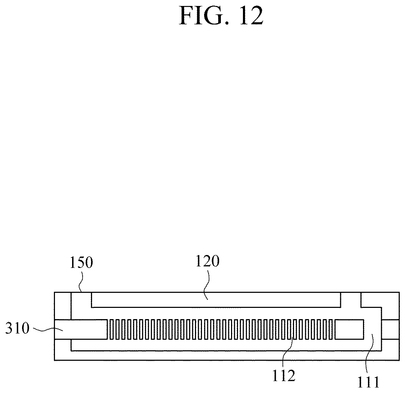

In yet another embodiment, as illustrated in FIG. 12, a chamber layer 310 may have the same configuration as the above example, except that the second passage 115 is omitted. If the outlet 150 is formed in the cover layer 120, an additional outlet may be formed in the bottom layer 130.

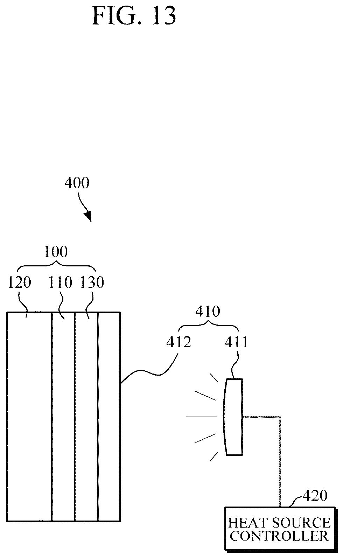

FIG. 13 is a block diagram illustrating an apparatus for gene amplification according to an embodiment of the present disclosure.

Referring to FIG. 13, an apparatus 400 for gene amplification includes the gene amplification chip 100, a heat source 410, and a heat source controller 420. The gene amplification chip 100 may have a configuration according to any of above example embodiments. The heat source 410 may heat the gene amplification chip 100 so that gene amplification may occur in the sample solution. For example, the heat source 410 may include a light source 411 and a photothermal film 412. The light source 411 may emit light onto a surface of the photothermal film 412 to heat the sample solution in the chamber layer 110.

The light source 411 may be any one of a light emitting diode (LED), vertical-cavity surface-emitting laser (VCSEL), a laser diode (LD), a tungsten lamp, a fluorescent lamp, a halogen lamp, a mercury lamp, a xenon lamp, and a metal-halide lamp, or a combination thereof. However, the light source 411 is not limited thereto. The light source 411 may emit light in a visible to infrared wavelength range, and a proper wavelength may be determined depending on the photothermal film 412.

The photothermal film 412, which is attached to an outer surface of the bottom layer 130, may convert light, emitted by the light source 411, into heat to heat the gene amplification chip 100. When the light source 411 emits light onto the surface of the photothermal film 412, the heat converted by the photothermal film 412 may be transferred rapidly to the sample solution, introduced in the chamber layer 110. The heat transfer occurs due to the bottom layer 130 and the chamber layer 110 having high thermal conductivity. Instead of providing the photothermal film 412 in the chamber layer 110 or the bottom layer 130, the photothermal film 412 is attached to the outer surface of the bottom layer 130, such that the light emitted by the light source 411 may be directly absorbed into the photothermal film 412, thereby improving light-to-heat conversion efficiency.

The photothermal film 412 having a predetermined thickness may be attached to the outer surface of the bottom layer 130. For example, the photothermal film 412 may be formed on the outer surface of the bottom layer 130 by patterning or deposition. In this case, the deposition may include chemical vapor deposition (CVD), physical vapor deposition (PVD), atomic layer deposition (ALD), sputtering, evaporation, etc., but is not limited thereto.

While it is illustrated that the photothermal film 412 is attached to the entire outer surface of the bottom layer 130, the photothermal film 412 may be attached to a portion of the outer surface of the bottom layer 130. The photothermal film 412 may have a thickness of 500 μm or less. However, the photothermal film 412 is not limited thereto, and may vary in consideration of heat conductivity and heat retention.

The photothermal film 412 may be made of a material, such as polymer, metal, metal oxide, nanocomposite, nanostructure, semiconductor, and the like. A polyimide (PI) film, having a relatively lower thermal conductivity but having a relatively higher heat retention than metal, may be used as the photothermal film 412. However, the photothermal film 412 is not limited thereto, and a gold (Au) film or an aluminum nanostructure (AINS) may also be used as the photothermal film 412.

The heat source controller 420 may control temperature and time of heating by the heat source 410. The heat source controller 420 may control the on/off, light intensity, light emission time, light emission period, and the like of the light source 411. In this case, the light intensity may be controlled by an intensity of an applied current or voltage. The controlling of the light emission time may include controlling an emission method such as pulse emission and continuous emission. The controlling of the light emission period may include controlling the on/off of the light source.

The heat source controller 420 controls the light source 411 to heat and cool the photothermal film 412, such that thermal cycling may occur to amplify genes in the chamber layer 110. In this case, the photothermal film 412 may be cooled by natural convection, and the like.

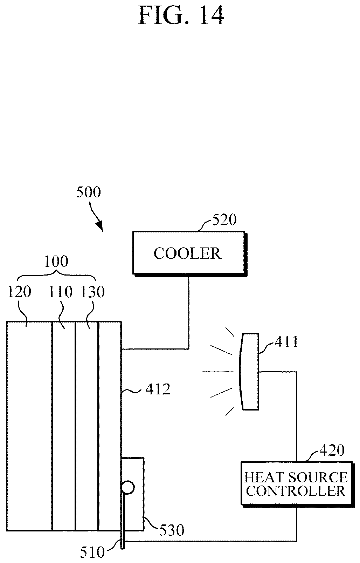

FIG. 14 is a block diagram illustrating an apparatus for gene amplification according to another embodiment of the present disclosure.

Referring to FIG. 14, an apparatus 500 for gene amplification may further include a temperature sensor 510 and a cooler 520. Here, the gene amplification chip 100, the heat source 410, and the heat source controller 420 may have the same configuration as the above embodiments. As described above, the photothermal film 412 may be attached to the outer surface of the bottom layer 130.

The temperature sensor 510 may measure temperature of the gene amplification chip 100. In this case, the temperature sensor 510 may include a thermocouple, an infrared sensor, etc., but is not limited thereto. The temperature sensor 510 may be disposed in a thermal pad 530 having high thermal conductivity, to be attached to the photothermal film 412 via the thermal pad 530, and may measure temperature of the photothermal film 412 converted by the light of the light source 411.

Alternatively, although not illustrated herein, the temperature sensor 510 may be disposed in the thermal pad 530 having high thermal conductivity to be attached to an outer surface of the bottom layer 130, on which the photothermal film 130 is not attached, via the thermal pad 530, and may measure temperature of the bottom layer 130 heated by heat conduction from the photothermal film 412 converted by the light of the light source 411.

The cooler 520 may cool the photothermal film 412. The cooler 520 may include a fan and the like. Based on the temperature measured by the temperature sensor 510, the heat source controller 420 may control the light source 411 according to a temperature profile. The temperature profile may be preset through experiments, and may include information, such as temperature over time, holding time, the number of times of repeating cycles, and the like. If the photothermal film 412 may be cooled by natural convection, the cooler 520 may be omitted.

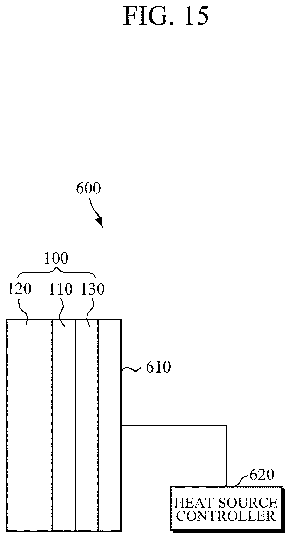

FIG. 15 is a block diagram illustrating an apparatus for gene amplification including a heat source according to another embodiment of the present disclosure.

Referring to FIG. 15, a heat source in an apparatus 600 for gene amplification may include a thermoelement 610. The gene amplification chip 100 may have the same configuration as the above embodiments. The thermoelement 610 is an element using Peltier effect. The Peltier effect is the phenomenon that a potential difference applied across an object causes a temperature difference between both ends of the object as heat is applied along with electrical current. That is, electrical current applied to an object causes a temperature difference, such that one side of the object is heated while the other side thereof is cooled.

The thermoelement 610 may be attached to the outer surface of the bottom layer 130. The heat source controller 620 controls the thermoelement 610 to heat and cool the chamber layer 110, such that thermal cycling may occur to amplify genes in the chamber layer 110. The heat source is not limited thereto, and various heat sources may be provided as long as the heat sources may perform the above functions.

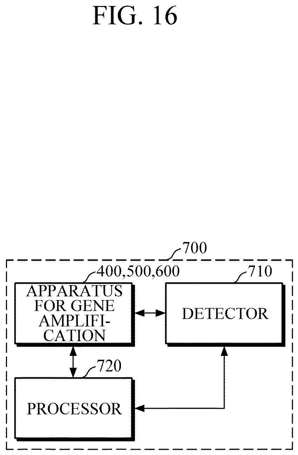

FIG. 16 is a block diagram illustrating an apparatus for bio-particle analysis according to an embodiment of the present disclosure.

Referring to FIG. 16, an apparatus 700 for bio-particle analysis may include one or more of the apparatuses 400, 500, and 600 for gene amplification, a detector 710, and a processor 720. The apparatuses 400, 500, and 600 for gene amplification may perform gene amplification in a sample solution, and may have the configuration as described in example embodiments.

The detector 710 may detect a signal generated in response to gene amplification occurring in solution introduced to the chamber layer of the gene amplification apparatus. The detector 710 may detect a signal generated in the apparatuses 400, 500, and 600 for gene amplification during or after the gene amplification performed by the apparatuses 400, 500, and 600 for gene amplification. In this case, the signal may include fluorescence signal, phosphor signal, absorbance signal, surface plasmon resonance signal, Raman signal, and the like.

The detector 710 may include a photomultiplier tube, a photo detector, a photomultiplier tube array, a photo detector array, a complementary metal-oxide semiconductor (CMOS) image sensor, etc., but is not limited thereto. In addition, the detector 710 may further include a filter for passing light of a specific wavelength, a mirror for directing the light radiating from the apparatuses 400, 500, and 600 for gene amplification, a lens for collecting light radiating from the apparatuses 400, 500, and 600 for gene amplification, and the like.

The processor 720 may analyze bio-particles based on the detected signal. The processor 720 may be electrically connected to the detector 710. In addition, the processor 720 may detect bio-particles by receiving and analyzing the signal detected by the detector 710. For example, the processor 720 may calculate a concentration of the sample solution by measuring the number of through holes 112 in which a fluorescence signal is observed in the apparatuses 400, 500, and 600 for gene amplification. The processor 720 may perform quantitative analysis of the bio-particles based on the detected signal using Poisson distribution, Surface-Enhanced Raman Spectroscopy (SERS), and the like based on the detected signal.

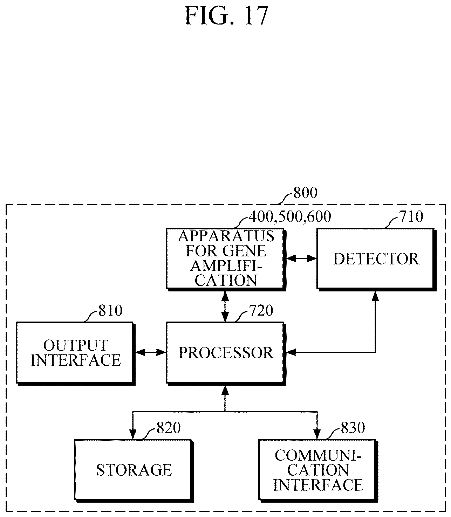

FIG. 17 is a block diagram illustrating an apparatus for bio-particle analysis according to another embodiment of the present disclosure.

Referring to FIG. 17, an apparatus 800 for bio-particle analysis may further include an output interface 810, a storage 820, and a communication interface 830. The apparatuses 400, 500, and 600 for gene amplification, the detector 710, and the processor 720 may be configured as described in example embodiments.

The output interface 810 may output processes or processing results, for example, a bio-particle analysis result of one or more of the apparatuses 400, 500, and 600 for gene amplification, the detector 710, and/or the processor 720. The output interface 810 may provide a user with information by visual, audio, and tactile methods and the like using a visual output module (e.g. display), an audio output module (e.g., speaker), a haptic module, and the like.

The storage 820 may store various data necessary for the apparatuses 400, 500, and 600 for gene amplification, the detector 710, and/or the processor 720, and/or processing results thereof. The storage 820 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The communication interface 830 may communicate with an external device to transmit and receive various data necessary for the detector 710 and/or the processor 720, and/or processing results thereof. In this case, the external device may be medical equipment, a printer to print out results, or a display device. In addition, the external device may be a digital TV, a desktop computer, a mobile phone, a smartphone, a tablet PC, a laptop computer, Personal Digital Assistants (PDA), Portable Multimedia Player (PMP), a navigation device, an MP3 player, a digital camera, a wearable device, etc., but is not limited thereto.

The communication interface 830 may communicate with the external device by using communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is merely exemplary and is not intended to be limiting.

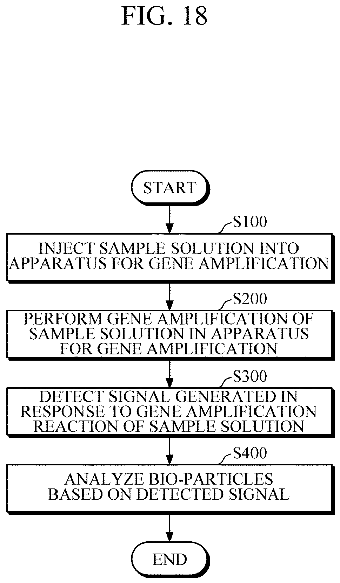

FIG. 18 is a flowchart illustrating an example of a method of bio-particle analysis.

First, a sample solution is injected through the inlet formed in the cover layer of the gene amplification chip in S100. In this case, a pretreatment process, such as heating, chemical treatment, treatment with magnetic beads, solid phase extraction, treatment with ultrasonic waves, etc., may be performed on the sample solution.

Then, when the sample solution is filled in the through holes of the chamber layer of the gene amplification chip, gene amplification is performed in the chamber layer in S200. In this case, if the sample solution is an RNA sample, reverse transcription of the RNA sample may be performed by using a reverse transcriptase. The enzyme reaction may include, for example, a nucleic acid amplification reaction including at least one of polymerase chain reaction (PCR) amplification and isothermal amplification, a redox reaction, a hydrolytic reaction, and the like. In this case, the heat source of the apparatus for gene amplification may heat the sample solution in the chamber layer. The heat source may perform thermal cycling for heating or cooling the chamber layer, to allow an amplification reaction of the sample solution to occur in the chamber layer of the gene amplification chip.

Subsequently, the detector of the apparatus for bio-particle analysis may detect a signal generated in response to the gene amplification reaction of the sample solution in the chamber layer of the gene amplification chip in S300. In this case, the signal may include fluorescence signal, phosphor signal, absorbance signal, surface plasmon resonance signal, Raman signal, and the like.

Next, the processor of the apparatus for gene amplification may analyze bio-particles based on the detected signal in S400. For example, the processor may analyze substance information and a level of amplification of the bio-particles and the like based on the detected signal by using Poisson distribution, Surface-Enhanced Raman Spectroscopy (SERS), and the like.

Example embodiments have been described herein with regard to several example embodiments. However, it will be obvious to those skilled in the art that various changes and modifications can be made without changing technical ideas and features of the present disclosure. Thus, it is clear that the above-described embodiments are illustrative in all aspects and are not intended to limit the present disclosure.

## Claims

1. A gene amplification chip comprising:

    a chamber layer formed with a first passage and through holes formed on one side of the first passage;
    a cover layer disposed on one side of the chamber layer, the cover layer formed with a cover channel communicating with the first passage and the through holes of the chamber layer, wherein the cover channel, the first passage and the through holes allow passage of liquids in a divided manner;
    a bottom layer disposed on another side of the chamber layer, the bottom layer formed with a bottom channel communicating with the first passage and the through holes of the chamber layer;
    wherein the cover layer is further formed with an inlet communicating with the cover channel; and
    at least one of the cover layer and the bottom layer is formed with an outlet communicating with any one of the cover channel and the bottom channel.

2. The gene amplification chip of claim 1, wherein the inlet in the cover layer is formed to be closer to the plurality of through hole than to the first passage of the chamber layer.

3. The gene amplification chip of claim 2, wherein the first passage and the cover channel are formed wherein a sample solution flows from the inlet to the cover channel to be stored in the plurality of through holes, and oil having a lower surface tension than the sample solution flows from the inlet to the cover channel to the first passage and to the bottom channel.

4. The gene amplification chip of claim 3, wherein the first passage has a larger cross-section than a cross-section of a cover channel region connected to the inlet.

5. The gene amplification chip of claim 4, wherein when the cross-section of the first passage is defined as a value obtained by multiplying a passage width by a passage length, and the cross-section of the cover channel region is defined as a value obtained by multiplying a channel height by a channel length, the passage length and the channel length are a same length in a same direction, and the passage width is greater than the channel height.

6. The gene amplification chip of one of claims 1 to 5, wherein the cover channel has a larger cross-section than a cross-section of the through holes
   and/or wherein a cross-section of the through holes has any one of a hexagonal shape, a tetragonal shape, a triangular shape, and a circular shape.

7. The gene amplification chip of one of claims 1 to 6, wherein the chamber layer further comprises a second passage formed on a side opposite the first passage with the through holes formed therebetween;
   in particular, wherein the outlet is formed to face the second passage.

8. The gene amplification chip of one of claims 1 to 7, wherein the outlet is formed in the cover layer;
   or

wherein the outlet is formed in the bottom layer.

9. The gene amplification chip of one of claims 1 to 8, wherein the chamber layer, the cover layer, and the bottom layer are separately formed and subsequently joined together;

or

wherein the chamber layer and the bottom layer are integrally formed with each other to be joined to the cover layer.

10. The gene amplification chip of one of claims 1 to 9, wherein each of the chamber layer, the cover layer, and the bottom layer are made of any one of silicon, metal, glass, and polymer;

and/or

wherein the cover layer is made of a transparent material.

11. The gene amplification chip of one of claims 2 to 5, wherein the first passage and the plurality of through holes in the chamber layer are separated by a non-through area, and cross sectional dimensions of the cover channel and the first passage are different from each other to flow liquid from the inlet through one of the first passage and the cover channel.

12. An apparatus for gene amplification, the apparatus comprising:

the gene amplification chip of one of claims 1 to 11, wherein the liquids are a sample solution;
a heat source configured to heat the gene amplification chip to allow gene amplification to occur in the sample solution; and
a heat source controller configured to control temperature and time of heating by the heat source.

13. The apparatus of claim 12, wherein the heat source comprises:

a light source configured to emit light; and
a photothermal film attached to an outer surface of the bottom layer and configured to convert light, emitted by the light source, into heat to heat the gene amplification chip.
and/or
wherein the heat source comprises a thermoelement.

14. An apparatus for bio-particle analysis, the apparatus comprising:

the apparatus for gene amplification of one of claims 12 to 13 in which gene amplification of the sample solution occurs;
a detector configured to detect a signal generated in response to the occurrence of gene amplification of the sample solution; and
a processor configured to analyze bio-particles based on the detected signal.

15. The apparatus of claim 14, wherein the processor calculates a concentration of the sample solution by measuring a number of through holes in which a fluorescence signal is observed.


**Amended claims in accordance with Rule 137(2) EPC.**

1. A gene amplification chip (100) comprising:

a chamber layer (110) formed with a first passage (111) and through holes (112) formed on one side of the first passage (111);
a cover layer (120) disposed on one side of the chamber layer (110), the cover layer formed with a cover channel (121) communicating with the first passage (111) and the through holes (112) of the chamber layer (110), wherein the cover channel (121), the first passage (111) and the through holes (112) allow passage of liquids in a divided manner;
a bottom layer (130) disposed on another side of the chamber layer (110), the bottom layer (130) formed with a bottom channel (131) communicating with the first passage (111) and the through holes (112) of the chamber layer (110);
wherein the cover layer (120) is further formed with an inlet (140) communicating with the cover channel (121); and

at least one of the cover layer (120) and the bottom layer (130) is formed with an outlet (150) communicating with any one of the cover channel (121) and the bottom channel (131).

2. The gene amplification chip (100) of claim 1, wherein the inlet (140) in the cover layer (120) is formed to be closer to the plurality of through hole (112) than to the first passage (111) of the chamber layer (110).

3. The gene amplification chip (100) of claim 2, wherein the first passage (111) and the cover channel (121) are formed wherein a sample solution may flow from the inlet (140) to the cover channel (121) to be stored in the plurality of through holes (112), and oil having a lower surface tension than the sample solution may flow from the inlet (140) to the cover channel (121) to the first passage (111) and to the bottom channel (131).

4. The gene amplification chip of claim 3, wherein the first passage has a larger cross-section than a cross-section of a cover channel region connected to the inlet.

5. The gene amplification chip (100) of claim 4, wherein when the cross-section of the first passage (111) is defined as a value obtained by multiplying a passage width (D) by a passage length ($L_1$), and the cross-section of the cover channel region is defined as a value obtained by multiplying a channel height (h) by a channel length ($L_2$), the passage length ($L_1$) and the channel length ($L_2$) are a same length in a same direction, and the passage width (D) is greater than the channel height (h).

6. The gene amplification chip (100) of one of claims 1 to 5, wherein the cover channel (121) has a larger cross-section than a cross-section of the through holes (112)
and/or wherein a cross-section of the through holes (112) has any one of a hexagonal shape, a tetragonal shape, a triangular shape, and a circular shape.

7. The gene amplification chip (100) of one of claims 1 to 6, wherein the chamber layer (110) further comprises a second passage (115) formed on a side opposite the first passage (114) with the through holes (112) formed therebetween;
in particular, wherein the outlet (150) is formed to face the second passage (115).

8. The gene amplification chip (100) of one of claims 1 to 7, wherein the outlet (150) is formed in the cover layer (120); or
wherein the outlet (150) is formed in the bottom layer (130).

9. The gene amplification chip (100) of one of claims 1 to 8, wherein the chamber layer (110), the cover layer (120), and the bottom layer (130) have been separately formed and subsequently joined together;
or
wherein the chamber layer (110) and the bottom layer (130) have been integrally formed with each other to be joined to the cover layer (120).

10. The gene amplification chip (110) of one of claims 1 to 9, wherein each of the chamber layer (110), the cover layer (120), and the bottom layer (130) are made of any one of silicon, metal, glass, and polymer;
and/or
wherein the cover layer (120) is made of a transparent material.

11. The gene amplification chip (100) of one of claims 2 to 5, wherein the first passage (111) and the plurality of through holes (112) in the chamber layer (110) are separated by a non-through area (114), and cross sectional dimensions of the cover channel (121) and the first passage (111) are different from each other to flow liquid from the inlet (140) through one of the first passage (111) and the cover channel (121).

12. An apparatus (400, 500, 600) for gene amplification, the apparatus (400, 500, 600) comprising:

the gene amplification chip (100) of one of claims 1 to 10;
a heat source (410) configured to heat the gene amplification chip (100) to allow gene amplification to occur in a sample solution; and
a heat source controller (420) configured to control temperature and time of heating by the heat source (410).

13. The apparatus (400, 500, 600) of claim 12, wherein the heat source (410) comprises:

a light source (411) configured to emit light; and
a photothermal film (412) attached to an outer surface of the bottom layer (130) and configured to convert light, emitted by the light source (411), into heat to heat the gene amplification chip (100)

and/or

wherein the heat source (410) comprises an element (610) using Peltier effect.

14. An apparatus (700) for bio-particle analysis, the apparatus (700) comprising:

the apparatus for gene amplification (400, 500, 600) of one of claims 12 to 13 in which gene amplification of the sample solution occurs;
a detector (710) configured to detect a signal generated in response to the occurrence of gene amplification of the sample solution; and
a processor (720) configured to analyze bio-particles based on the detected signal.

15. The apparatus (700) of claim 14, wherein the processor (720) is configured to calculate a concentration of the sample solution by measuring a number of through holes (112) in which a fluorescence signal is observed.

# FIG. 1

# FIG. 2

# FIG. 3

FIG. 4

# FIG. 5

# FIG. 6

# FIG. 7A

# FIG. 7B

FIG. 7C

E₂

# FIG. 8A

FIG. 8B

# FIG. 8C

# FIG. 8D

# FIG. 8E

FIG. 8F

# FIG. 8G

FIG. 9A

FIG. 9B

FIG. 9C

FIG. 9D

10

# FIG. 10A

FIG. 10B

# FIG. 10C

# FIG. 10D

# FIG. 11

# FIG. 12

# FIG. 13

# FIG. 14

# FIG. 15

600

100

120 110 130

610

620

HEAT SOURCE
CONTROLLER

FIG. 16

FIG. 17

# FIG. 18

```
        ┌─────────┐
        │  START  │
        └─────────┘
             │
             ▼                    ⟋S100
┌─────────────────────────────────────────┐
│        INJECT SAMPLE SOLUTION INTO       │
│    APPARATUS FOR GENE AMPLIFICATION      │
└─────────────────────────────────────────┘
             │
             ▼                    ⟋S200
┌─────────────────────────────────────────┐
│     PERFORM GENE AMPLIFICATION OF        │
│     SAMPLE SOLUTION IN APPARATUS         │
│        FOR GENE AMPLIFICATION            │
└─────────────────────────────────────────┘
             │
             ▼                    ⟋S300
┌─────────────────────────────────────────┐
│      DETECT SIGNAL GENERATED IN          │
│   RESPONSE TO GENE AMPLIFICATION         │
│    REACTION OF SAMPLE SOLUTION           │
└─────────────────────────────────────────┘
             │
             ▼                    ⟋S400
┌─────────────────────────────────────────┐
│         ANALYZE BIO-PARTICLES            │
│        BASED ON DETECTED SIGNAL          │
└─────────────────────────────────────────┘
             │
             ▼
        ┌─────────┐
        │   END   │
        └─────────┘
```

Europäisches
Patentamt
European
Patent Office
Office européen
des brevets

# EUROPEAN SEARCH REPORT

Application Number

EP 23 15 6797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/191098 A1 (BEARD NIGEL P [US] ET AL) 30 July 2009 (2009-07-30) * paragraphs [0143], [0164], [0215] – [0217]; figures 20-22 * | 1-15 | INV. B01L3/00 B82Y5/00 C12Q1/6844 G01N21/64 |
| X | US 2010/178208 A1 (XIAO CAIBIN [US] ET AL) 15 July 2010 (2010-07-15) * paragraphs [0050] – [0055]; figure 1 * | 1-15 | |
| A | EP 3 142 790 A1 (UNIV LIMERICK [IE]) 22 March 2017 (2017-03-22) * abstract; figures 1-14 * | 1-15 | |

TECHNICAL FIELDS
SEARCHED (IPC)

B01L
B82Y
G01N
C12Q

The present search report has been drawn up for all claims

| Place of search | Date of completion of the search | Examiner |
|---|---|---|
| The Hague | 8 August 2023 | Viskanic, Martino |

EPO FORM 1503 03.82 (P04C01)

## EP 4 338 841 A1

### ANNEX TO THE EUROPEAN SEARCH REPORT
### ON EUROPEAN PATENT APPLICATION NO.

EP 23 15 6797

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2023

| Patent document cited in search report | | Publication date | Patent family member(s) | | Publication date |
|---|---|---|---|---|---|
| US 2009191098 | A1 | 30-07-2009 | EP | 2010326 A2 | 07-01-2009 |
| | | | US | 2006233671 A1 | 19-10-2006 |
| | | | US | 2009191098 A1 | 30-07-2009 |
| | | | WO | 2007117445 A2 | 18-10-2007 |
| US 2010178208 | A1 | 15-07-2010 | CN | 101297189 A | 29-10-2008 |
| | | | US | 2007092407 A1 | 26-04-2007 |
| | | | US | 2007092411 A1 | 26-04-2007 |
| | | | US | 2010178208 A1 | 15-07-2010 |
| EP 3142790 | A1 | 22-03-2017 | CA | 2948975 A1 | 19-11-2015 |
| | | | CA | 2948976 A1 | 19-11-2015 |
| | | | CA | 2948979 A1 | 19-11-2015 |
| | | | EP | 3142790 A1 | 22-03-2017 |
| | | | EP | 3142791 A1 | 22-03-2017 |
| | | | EP | 3142792 A2 | 22-03-2017 |
| | | | EP | 3539662 A1 | 18-09-2019 |
| | | | US | 2017080417 A1 | 23-03-2017 |
| | | | US | 2017080426 A1 | 23-03-2017 |
| | | | US | 2017216838 A1 | 03-08-2017 |
| | | | WO | 2015173651 A1 | 19-11-2015 |
| | | | WO | 2015173652 A1 | 19-11-2015 |
| | | | WO | 2015173658 A2 | 19-11-2015 |

EPO FORM P0459

For more details about this annex : see Official Journal of the European Patent Office, No. 12/82